# EUROPEAN PATENT SPECIFICATION

(11) **EP 1 632 189 B1**
(45) Date of publication and mention of the grant of the patent: **21.10.2009**
(21) Application number: 05018587.5
(22) Date of filing: 26.08.2005
(51) Int. Cl.: A61B 17/22

(54) **Wire for removing intravascular foreign body and medical instrument**
Draht zur Entfernung intravaskulärer Fremdkörper und medizinisches Instrument
Fil vasculaire servant à enlever un corps étranger et un instrument médical

(30) Priority: 07.09.2004 JP 2004260097
(43) Date of publication of application: 08.03.2006
(73) Proprietor: TERUMO KABUSHIKI KAISHA, Tokyo (JP)
(72) Inventor: Kanamaru, Takeshi, Terumo Kabushiki Kaisha, Ashigarakami-gun Kanagawa 259-0151 (JP)
(74) Representative: TBK-Patent

(56) References cited:
- US-A1- 2002 072 764
- US-A1- 2003 135 233
- US-A1- 2004 167 567
- US-B1- 6 416 519

## Description

### BACKGROUND OF THE INVENTION

The present invention relates to a wire for removing an intravascular foreign body (such as embolus in a vessel) and a medical instrument.

The vital statistics of population published by the Ministry of Health, Labor, and Welfare indicates that cancer dominates in the cause of Japanese death and heart disease and cerebral apoplexy come second and third. The increasing deaths and sequelae due to cerebral apoplexy urgently demand to establish its therapeutic method.

A recent development in therapy of cerebral apoplexy is thrombolysis that employs a thrombolytic agent to cure brain infarction in its acute phase. It is effective but its effectiveness is limited. That is, the thrombolytic agent takes a long time for thrombolysis or produces smaller thrombi that scatter to form new emboli. In addition, it has been found that some emboli are insoluble by treatment with a thrombolytic agent.

It has been proved in the U.S. and Europe that the probability to save lives and reduce sequelae would be high if the blood flow is resumed within 3 hours after the onset of cerebral apoplexy. Thus, there is a strong demand for development of a new medical instrument that can be inserted into a cerebral vessel to remove the thrombus directly. An example of such a medical instrument is a wire for removing an intravascular foreign body, which is disclosed in Japanese Patent Laid-open No. 2004-16668. A system for removing an obstruction from a blood vessel including an obstruction engaging element and an expandable capture element is described in US 2002/007264.

The wire for removing an intravascular foreign body consists of a wire proper, two branch wires (which branch out from the wire proper), and a plurality of filaments connecting the branch wires. The branch wires and the filaments form a space in which an intravascular foreign body is captured.

The disadvantage of the wire for removing an intravascular foreign body mentioned above is that it sometimes fails to capture an intravascular foreign body depending on its size.

For example, if the intravascular foreign body is smaller than the space in which it is to be captured, it might slip off through the gap between the filaments. In this case, it would be necessary to exchange the wire for removing an intravascular foreign body that matches the size of the foreign body to be captured. This is troublesome.

### SUMMARY OF THE INVENTION

It is desirable for the present invention to provide a wire to surely capture and remove an intravascular foreign body. It is another object of the present invention to provide a medical instrument.

The above-mentioned object of the present invention is achieved by a wire comprising the features of the attached claim 1. Further developments are the subject matter of the depending claims. A medical instrument according to the present invention is stated in claim 8. In particular, the present invention discloses:
(1) A wire for removing an intravascular foreign body which includes a flexible long wire body, a capturing part which is arranged on the forward end of the wire body and in which is formed a space to capture an intravascular foreign body, and an auxiliary wire which is arranged on the forward end of the wire body, the auxiliary wire assuming a first state in which it projects into the space and a second state in which it retreats from the space as it moves relative to the capturing part.
(2) The wire for removing an intravascular foreign body as defined in (1) above, in which the wire body is composed of a tube and a linear body that passes through the tube for its movement relative to the tube, and in which the auxiliary wire is arranged on the forward end of the tube and the capturing part is arranged on the forward end of the linear body.
(3) The wire for removing an intravascular foreign body as defined in (1) or (2) above, in which the auxiliary wire is arranged on that part which is closer to the base end than the capturing part.
(4) The wire for removing an intravascular foreign body as defined in any of (1) to (3) above, in which the auxiliary wire in the first state is in contact with the foreign body and has an expanded part which expands toward the forward end.
(5) The wire for removing an intravascular foreign body as defined in (4) above, in which the expanded part assumes a helical shape.
(6) The wire for removing an intravascular foreign body as defined in (5) above, in which the loop diameter of the helical expanded part gradually increases in going toward the forward end.
(7) The wire for removing an intravascular foreign body as defined in any of (1) to (6) above, which has a means for limiting the maximum extent to which the auxiliary wire projects in the first state.
(8) A medical instrument which includes the wire for removing an intravascular foreign body as defined in any of (1) to (7) above and a catheter having a lumen that receives therein the wire for removing an intravascular foreign body.

According to the present invention, the desire of removing an intravascular foreign body is achieved surely owing to the auxiliary wire that helps capture a foreign body.

In a modified embodiment, the auxiliary wire has an expanded part so that a foreign body is held between the capturing part and the expanded part. This ensures the capture and removal of a foreign body.

### BRIEF DESCRIPTION OF THE DRAWINGS

Fig. 1 is a plan view (with a partial longitudinal sectional view) showing the wire for removing an intravascular foreign body (in the second state) pertaining to the first embodiment of the present invention.
Fig. 2 is a plan view (with a partial longitudinal sectional view) showing the wire for removing an intravascular foreign body (in the first state) which is shown in Fig. 1.
Fig. 3 is a side view showing the wire for removing an intravascular foreign body which is shown in Fig. 1.
Fig. 4 is a diagram illustrating how to use the wire for removing an intravascular foreign body which is shown in Fig. 1.
Fig. 5 is a diagram illustrating how to use the wire for removing an intravascular foreign body which is shown in Fig. 1.
Fig. 6 is a diagram illustrating how to use the wire for removing an intravascular foreign body which is shown in Fig. 1.
Fig. 7 is a diagram illustrating how to use the wire for removing an intravascular foreign body which is shown in Fig. 1.
Fig. 8 is a diagram illustrating how to use the wire for removing an intravascular foreign body which is shown in Fig. 1.
Fig. 9 is a plan view (with a partial longitudinal sectional view) showing the vicinity of the capturing part of the wire for removing an intravascular foreign body (in the first state) pertaining to the second embodiment of the present invention.
Fig. 10 is a plan view (with a partial longitudinal sectional view) showing the vicinity of the capturing part of the wire for removing an intravascular foreign body (in the first state) pertaining to the third embodiment of the present invention.
Fig. 11 is a plan view (with a partial longitudinal sectional view) showing the vicinity of the capturing part of the wire for removing an intravascular foreign body (in the second state) pertaining to the third embodiment of the present invention.
Fig. 12 is a side view showing the wire for removing an intravascular foreign body which is shown in Fig. 11.

### DETAILED DESCRIPTION OF THE PREFERRED EMBODIMENTS

A detailed description is given below of the wire for removing an intravascular foreign body and the medical instrument of the present invention with reference to the preferred embodiments shown in the accompanying drawings.

### First Embodiment

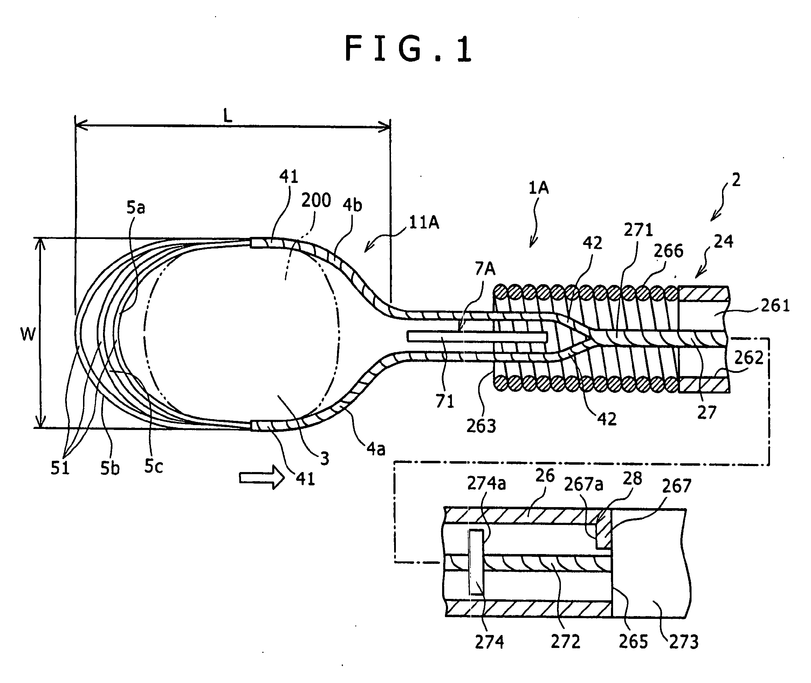

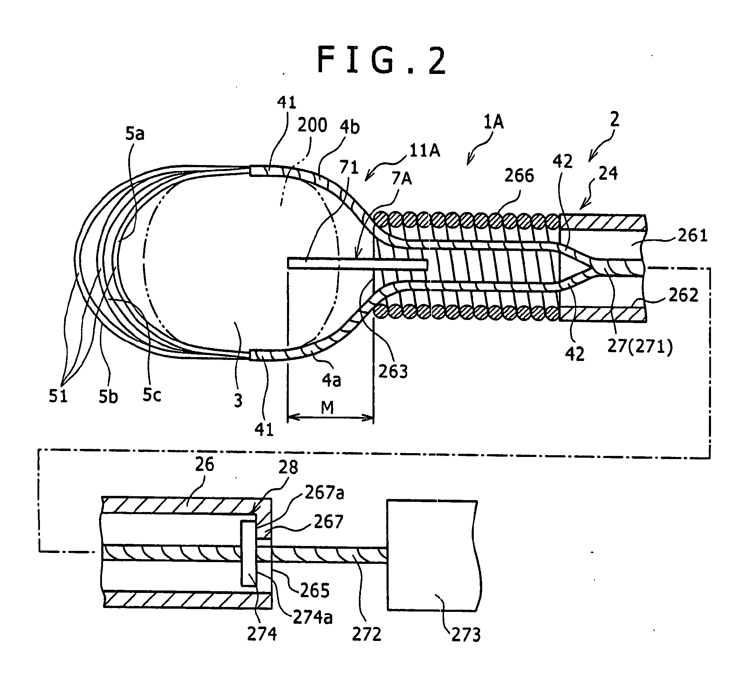

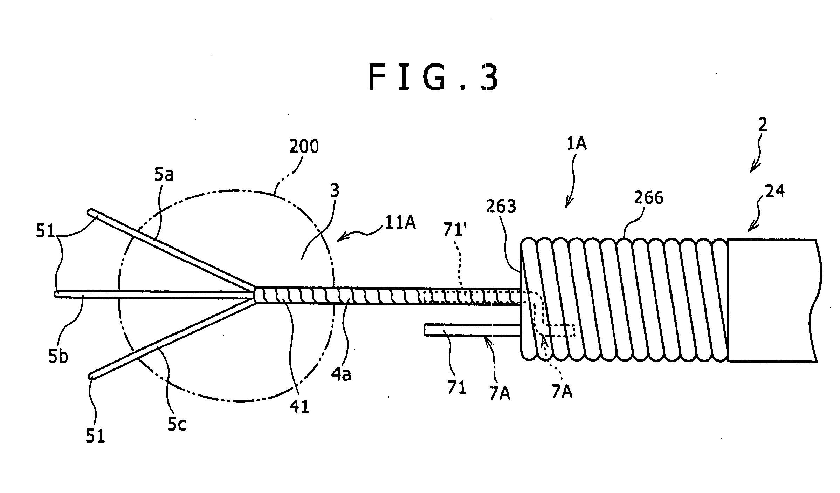

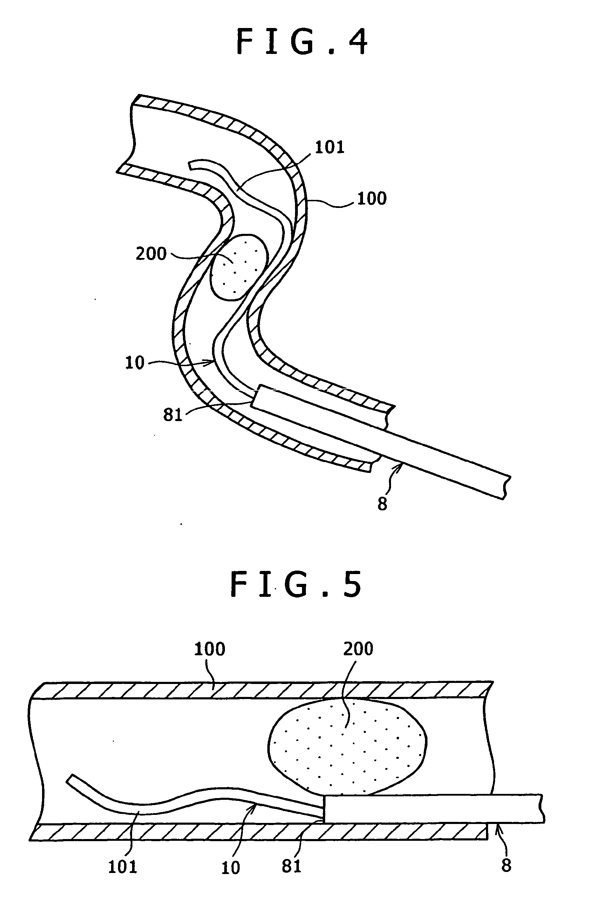

Fig. 1 is a plan view (with a partial longitudinal sectional view) showing the wire for removing an intravascular foreign body (in the second state) pertaining to the first embodiment of the present invention. Fig. 2 is a plan view (with a partial longitudinal sectional view) showing the wire for removing an intravascular foreign body (in the first state) which is shown in Fig. 1. Fig. 3 is a side view showing the wire for removing an intravascular foreign body which is shown in Fig. 1. Figs. 4 to 8 are respectively diagrams illustrating how to use the wire for removing an intravascular foreign body which is shown in Fig. 1. In the following description the right side and left side of Figs. 1 to 8 are designated as "base end (proximal end)" and "forward end (distal end)", respectively.

Fig. 1 shows the wire 1A for removing an intravascular foreign body, which is intended to capture (or pinch) and remove a foreign body (such as thrombus and clot) which causes embolism in the vessel 100. A foreign body will be referred to as "embolus 200" hereinafter.

The wire 1A to remove an intravascular foreign body consists of a long wire body 2, a capturing part 11A arranged on the forward end of the wire body 2, an auxiliary wire 7A arranged on the forward end of the wire body 2, and a means 28 for limiting the maximum extent to which the auxiliary wire 7A projects.

Each part is constructed as described in the following.

The wire body 2 shown in Fig. 1 consists of a tube 26 and a linear body 27 which passes through the inside 261 of the tube 26. The wire body 2 has adequate rigidity and resilience (flexibility) over its entire length.

The tube 26 has a coil 266 arranged on (connected to) the forward end thereof. The coil 266 makes the forward end of the tube 26 (including the coil 266) more resilient (or flexible).

As shown in Figs. 1 and 2, the linear body 27 is constructed such that it is movable relative to the tube 26. The linear body 27 is also constructed such that its base 272 projects from the base-end opening 265 of the tube 26. See Fig. 2.

The linear body 27 has a manipulating member 273 attached to its base end 272, which is designed to manipulate (move) the linear body 27 in its lengthwise direction.

The manipulating member 273 permits the linear body 27 to be grasped easily at the time of its handling.

The parts constructing the wire body 2 may be formed from any materials without specific restrictions, such as metallic and plastic materials, which may be used alone or in combination.

The wire body 2 may vary in length depending on the position and size of the vessel 100 to which it is applied. A preferred length ranges from 500 to 4000 mm usually, and more preferred length ranges from 1500 to 2200 mm.

The wire body 2 (or the tube 26) may also vary in thickness (outside diameter) depending on the position and size of the vessel 100 to which it is applied. A preferred outside diameter is usually 0.1 to 2.0 mm on average, and more preferred length ranges from 0.25 to 0.9 mm.

The wire body 2 (or the tube 26) should preferably consist of a first part (which is comparatively hard and is placed at the base end), a third part (which is comparatively soft and is placed at the forward end), and a second part (which is variable in flexibility and is placed at the intermediate position between the first part and third part). In other words, the wire body 2 should preferably be formed in such a way that it gradually decreases in rigidity (flexural and torsional rigidity) in going from its base end to its forward end. The gradually changing rigidity permits the manipulation by hands to be certainly transmitted to the forward end 24 (including the coil 266) of the wire body 2. With such properties (that make the forward end 24 flexible), the wire body 2 easily proceeds and bends in the vessel 100 without damaging the vessel 100. Such properties permit the wire body 2 to maintain its torque transmission and its pushabiliy while preventing kink resistance(or flexing). This contributes to higher safety.

The wire body 2 (or the tube 26) may have a coating layer on its outer surface for reduction of frictional with the inside of the catheter 8 (mentioned later). The coating layer permits smooth insertion into and removal from the catheter. The coating layer may be formed from a fluorocarbon resin (such as polytetrafluoroethylene or "Teflon") or a hydrophilic polymer which becomes lubricous in a wet condition.

The linear body 27 may also have a coating layer on its outer surface (as in the case of the tube 26) for reduction of friction with the internal surface 262 of the tube 26. The coating layer produces the same effect as mentioned above.

As shown in Figs. 1 to 3, the capturing part 11A consists of two branch wires 4a and 4b (branching out from the forward end 271 of the linear body 27 (or the forward end of the wire body 2) and a plurality (three in this embodiment) of filaments 5a, 5b, and 5c connecting the two branch wires 4a and 4b.

Both the base end 42 of the branch wire 4a and the base end 42 of the branch wire 4b are fixed to the forward end 271 of the linear body 27. Fixing may be accomplished in any manner without specific restrictions. One way is to wind the bases of the branch wires 4a and 4b around the forward end 271 of the linear body 27 and bond them together by brazing, welding, or adhesion.

The three filaments 5a, 5b, and 5c extend from the forward end 41 of the branch wire 4a to the forward end 41 of the branch wire 4b.

The filaments 5a, 5b, and 5c may be fixed to the branch wires 4a and 4b in any manner without specific restrictions. The method for fixing may include brazing, welding, and adhesion. The branch wires 4a and 4b may be a stranded wire composed of a plurality of linear bodies. In this case, the filaments 5a, 5b, and 5c may be fixed to the branch wires 4a and 4b by tangling in the stranded wire. An alternative way is that the individual linear bodies of the branch wires 4a and 4b are extended so that the extended parts function as the filaments 5a, 5b, and 5c, and the branch wires 4a and 4b and the filaments 5a, 5b, and 5c are made into one body. This method is simple but achieves strong connection between the filaments 5a, 5b, and 5c and the branch wires 4a and 4b.

These filaments 5a, 5b, and 5c are fixed to the forward end 41 of the branch wire 4a and the forward end 41 of the branch wire 4b in such a way that their central parts expand forward. In other words, the branch wires 4a and 4b and the filaments 5a, 5b, and 5c form an ellipse as shown in Fig. 1 (which is a plan view). In this embodiment, the forward end of the filaments 5a, 5b, and 5c smoothly curve so that they do not damage the internal wall of the vessel 100. This configuration ensures higher safety.

As shown in Fig. 3, the filament 5b is in the plane (perpendicular to the paper of Fig. 2) through which the extension of the central axis of the wire body 2 (the linear body 27) passes. In other words, the filament 5b overlaps with the extension of the central axis of the wire body 2 in Fig. 2 (which is a side view). The filaments 5a and 5c are inclined such that they get far from the extension of the central axis of the wire body 2 as they extend forward. In other words, the filament 5a slopes up leftward and the filament 5c slopes down leftward in Fig. 3 (which is a side view). As mentioned above, the tops 51 of the filaments 5a, 5b, and 5c are apart from each other.

In the capturing part 11A is formed the foreign body capturing space 3 (in which the embolus 200 is captured) which is surrounded by the branch wires 4a and 4b and the filaments 5a, 5b, and 5c. See Figs. 1 and 2. In other words, the filaments 5a, 5b, and 5c form the foreign body capturing part which assumes a basket-like shape.

The capturing part 11A constructed in this way permits the foreign body capturing space 3 to capture (hold) the embolus 200 from either the upper side or the lower side (shown in Fig. 2). This facilitates the capturing of the embolus 200.

The capturing part 11A constructed in this way is not specifically restricted in the outside diameter of the branch wires 4a and 4b. The outside diameter should preferably be 0.05 to 0.9 mm, more preferably 0.1 to 0.5 mm. Incidentally, the outside diameter of the branch wires 4a and 4b denotes the overall outside diameter in the case of the stranded wire.

The filaments 5a, 5b, and 5c are not specifically restricted in outside diameter. The outside diameter should preferably be 0.025 to 0.2 mm, more preferably 0.05 to 0.1 mm.

According to the present invention, the size of the capturing part 11A is not specifically restricted. It varies depending on the thickness of the vessel to be treated. The overall length (indicated by L in Fig. 1) of the capturing part 11A in its expanded state should preferably be 2 to 40 mm, more preferably 4 to 20 mm. The overall width (indicated by W in Fig. 1) of the capturing part 11A in its expanded state should preferably be 1 to 30 mm, more preferably 2 to 5 mm.

For use in the cerebral artery end (M1 portion), the capturing part 11A should have an overall length (indicated by L in Fig. 1) of at least 7 mm, preferably 8 to 15 mm, and an outside diameter (indicated by W in Fig. 1) of 2 to 5 mm, in its expanded state. This is because the inside diameter of the vessel in the cerebral artery end (M1 portion), where brain infarction occurs frequently, is about 3 to 4 mm and, according to doctors' experience, the size of thrombus causing embolus is about 3 mm in outside diameter and about 7 mm in length in many cases.

The capturing part 11A is capable of decreasing in diameter so that it can pass through (or can be inserted into) the lumen of the catheter 8. According to the present invention, the capturing part 11A is comparatively simple in structure, so that it can be made thin easily.

If the wire for removing an intravascular foreign body is to be inserted into a microcatheter, the outside diameter (indicated by W in Fig. 1) of the capturing part 11A in its contracted state should preferably be no larger than 0.53 mm (0.021 inch), more preferably no larger than 0.46 mm (0.018 inch).

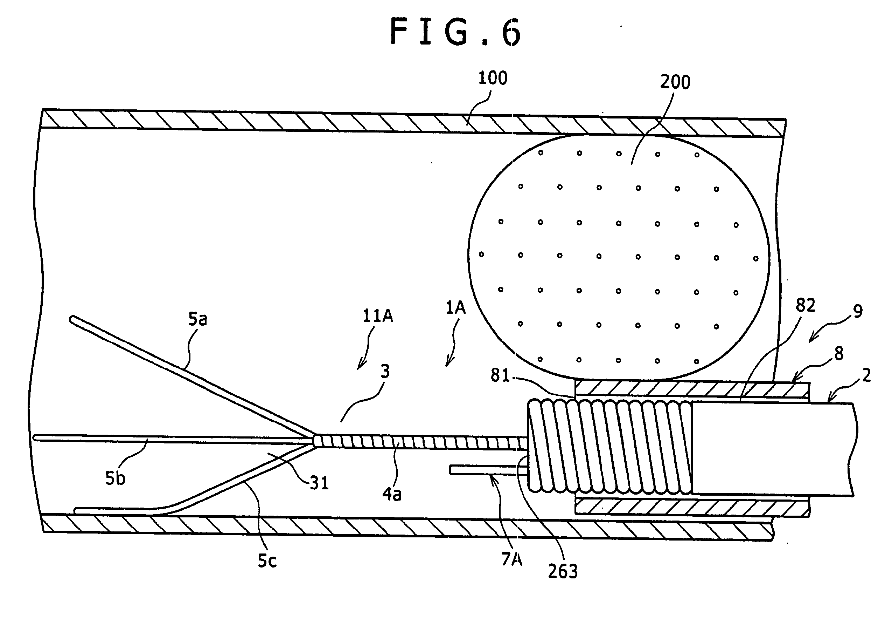

The capturing part 11A returns to its expanded state from its contracted state by its own resilience as it is pushed out (or exposed) from the forward opening 81 of the catheter 8. This takes place when the wire 1A for removing an intravascular foreign body is advanced (moved relative to the catheter). See Fig. 6.

Thus, the capturing part 11A changes in its shape (expanded state and contracted state) automatically as it is pushed out from and pulled back into the forward opening 81 of the catheter 8.

Incidentally, the capturing part 11A (including the branch wires 4a and 4b and the filaments 5a, 5b, and 5c) should preferably be formed from any radiopaque material which is not specifically restricted. It includes, for example, gold, platinum, platinum-iridium alloy, tungsten, tantalum, palladium, lead, silver, and their alloys and compounds. Such radiaopaque materials facilitate manipulation (to capture the embolus 200 by the capturing part 11A) with the help of X-ray radioscopy and the like.

The capturing part 11A may be formed from various metallic materials and plastic (mono- or multi-) filaments. Metallic materials include stainless steel (such as SUS 304), β-titanium steel, Co-Cr alloy, piano wire, platinum-iridium alloy (Pt₉₀-Ir₁₀, Pt₈₀-Ir₂₀), noble metal alloy, nickel-titanium alloy, and other alloys having springy properties.

Of these metallic materials for the capturing part 11A, the one which exhibits superelasticity in the living organism is preferable. It surely changes from contracted state to expanded state, and vice versa.

The alloy that exhibits superelasticity in the living organism denotes the one which restores nearly to its original shape after deformation (bending, stretching, and compressing) to an extent at which ordinary metals undergo plastic deformation at the living body temperature (about 37°C). It includes so-called shape memory alloy and superelastic alloy.

The shape memory alloy and superelastic alloy are specifically restricted. Titanium alloys (Ti-Ni, Ti-Pd, Ti-Nb-Sn) and copper alloys are preferable. The titanium alloys should preferably be composed of 30-52 atom% of titanium, with the remainder being nickel and optional elements (more than 10 atom%). The optional elements, which are not specifically restricted, may be selected from iron, cobalt, platinum, palladium, and chromium (each less than 3 atom%), and copper and vanadium (each less than 10 atom%).

Preferred superelastic alloys are those which undergo transformation from austenite phase at normal temperature or body temperature (about 37°C) into martensite phase under stress.

The surface of the capturing part 11A should be provided with a means for preventing the embolus 200 (which has been captured) from slipping off from the capturing part 11A. Such an anti-slipping means increases friction between the capturing part 11A and the embolus 200, thereby allowing the capturing part 11A to surely hold (capture) the embolus 200.

The anti-slipping means is not specifically restricted. It may be formed by coating with an elastic material (such as rubber) having a comparative high coefficient of friction or by sand blasting which produces fine rough surfaces or surface irregularities.

Another anti-slipping means may be fiber winding or flocking, which increases the surface area of the capturing part 11A and hence increases the area which comes into contact with the embolus 200. In this way, it is possible to increase friction between the capturing part 11A and the embolus 200, as in the case of the above-mentioned anti-slipping means, thereby ensuring the capturing of the embolus 200.

As shown in Fig. 1, the auxiliary wire 7A is a nearly straight linear body. It is fixed to the forward end of the inside 262 (or the inside of the coil 266) defining the internal space 261 of the tube 26, such that it is approximately parallel to the lengthwise direction of the tube 26. No specific restrictions are imposed on the method for fixing the auxiliary wire 7A to the tube 26. Fixing may be accomplished by welding (brazing) or adhesion. Incidentally, the auxiliary wire 7A may be a solid wire or a hollow wire.

The auxiliary wire 7A has its forward end 71 projecting from the forward opening 263 of the tube 26 toward the capturing part 11A. In other words, the auxiliary wire 7A is closer to the base end than to the capturing part 11A.

The auxiliary wire 7A placed in the tube 26 (or the wire 1A for removing an intravascular foreign body) as mentioned above permits the capturing part 11A to easily approach the auxiliary wire 7A as the manipulating part 273 is pulled toward the base end (in the arrow direction in Fig. 1).

The auxiliary wire 7A may be formed from the same superelastic alloy as listed above for the capturing part 11A. Thus, the auxiliary wire 7A may deform under external force and restore its shape (straight) in the absence of external force.

The surface of the auxiliary wire 7A may be provided with an anti-slipping means as mentioned above for the capturing part 11A. The anti-slipping means will surely prevent the captured embolus 200 from slipping off from the auxiliary wire 7A as in the case of the capturing part 11A.

The wire 1A for removing an intravascular foreign body, which is constructed as mentioned above, permits the capturing part 11A to move relative to the auxiliary wire 7A (come close or get away) as the manipulating part 273 is pulled toward the base end. Thus, the auxiliary wire 7A assumes a first state (in which it projects into the foreign body capturing space 3 or the capturing part 11A as referred to Fig. 2) and a second state (in which it retreats from the foreign body capturing space 3 as referred to Fig. 1). In other words, the auxiliary wire 7A freely gets in or gets out of the foreign body capturing space 3.

The embolus 200 which has been captured in the foreign body capturing space 3 while the auxiliary wire 7A is in the second state is stabbed with the auxiliary wire 7A when the auxiliary wire assumes the first state. See Figs. 1 and 2. In this way, it is possible to prevent the embolus 200 from slipping off from the foreign body capturing space 3, and hence it is possible to surely capture the embolus 200. And, eventually, it is possible to surely remove the embolus 200 from the vessel 100.

Incidentally, as shown in Fig. 3, the auxiliary wire 7A may be properly bent at its middle so that its forward end 71' approximately coincides with the central axis of the wire body 2. (The auxiliary wire 7A is indicated by broken line in Fig. 3.)

As shown in Fig. 2, the regulating means 28 regulates the maximum length over which the auxiliary wire 7A in the first state projects into the foreign body capturing space 3.

The regulating means 28 consists of a projecting part 267 and an expanded part 274. The projecting part 267 projects in the radial direction from the inside 262 in the neighborhood of the base opening 265 of the tube 26. The expanded part 274 is a part which expands in the radial direction from the outside of the linear body 27 in the internal space 261 of the tube 26.

The expanded part 274 is so formed as to fix a ring-shaped member to the linear body 27.

The projecting part 267 of the tube 26 and the expanded part 274 of the linear body 27 are constructed such that the face 267a at the forward end side of the projecting part 267 comes into contact with the face 274a at the base end side of the expanded part 274 when the auxiliary wire 7A assumes the first state (or when the linear body 27 moves toward the base end relative to the tube 26). This prevents the linear body 27 moves excessively toward the base end. When they are in contact with each other, the distance (the length indicated by M in Fig. 2) becomes the maximum length of projection.

The regulating means 28 constructed as mentioned above protects the embolus 200 from being stabbed excessively by the auxiliary wire 7A. In this way, it is possible to prevent the embolus 200 from being broken by the auxiliary wire 7A.

Incidentally, the maximum length M of projection is not specifically restricted. It should preferably be shorter than the overall length of the capturing part 11A in its expanded state, but it depends on the length L of the capturing part 11A. It should preferably be 2 to 20 mm, more preferably 3 to 6 mm.

The expanded part 274 may be fixed to the linear body 27, with its position properly adjusted. This adjustment adjusts the maximum length M of projection. In this way, it is possible to set up the maximum length M of projection according to the size (length) of the embolus 200.

The number of the branch wires is not limited to two; it may be three or more.

The number of filaments is not limited to three; it may be two or four or more.

The number of the auxiliary wire 7A is not limited to one; it may be two or more.

The medical instrument 9 according to the present invention consists of the wire 1A for removing an intravascular foreign body and the catheter 8 which has the lumen 82 formed therein.

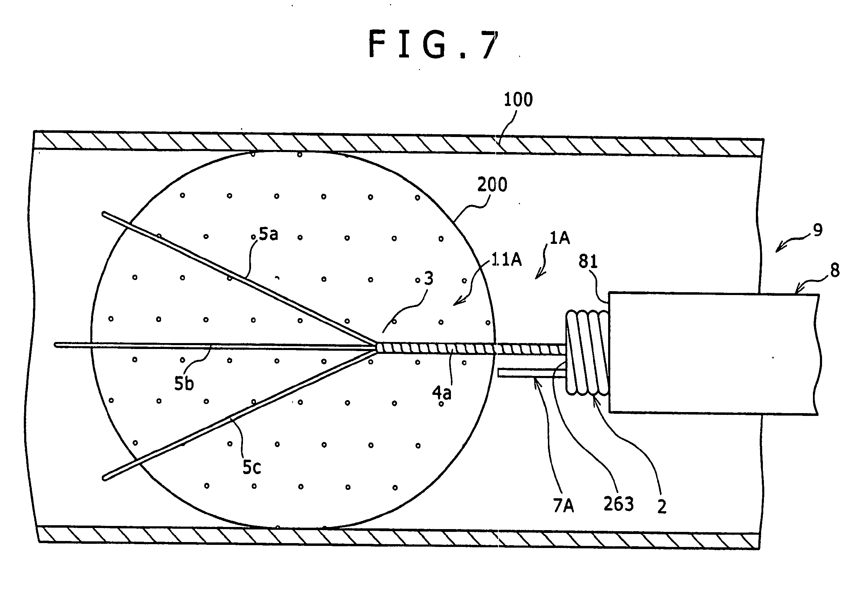

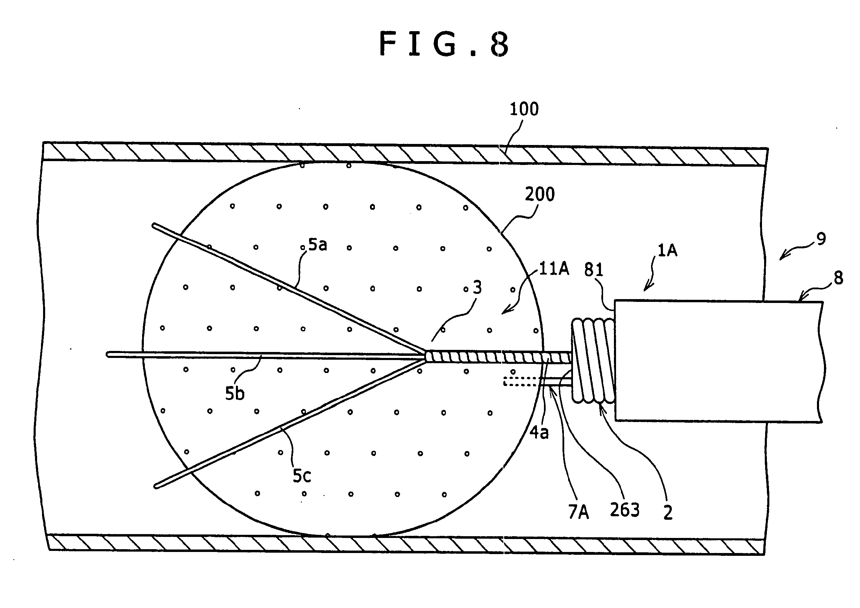

A detailed description is given below of one way of using the wire 1A for removing an intravascular foreign body.
[1] Fig. 4 depicts the vessel 100 which is clogged with the embolus 200 (such as thrombus) and inhibited the blood flow. The embolus 200 is almost immobile because it is pushed against the inner wall of the vessel 100 by blood pressure. It is assumed that the presence of the embolus 200 is previously confirmed by radioscopy.
   The first step is to insert the catheter (microcatheter) 8 and the guide wire 10 (which has been passed through the lumen 82 of the catheter 8) into the vessel 100. The second step is to project the guide wire 10 from the forward open end 81 of the catheter 8 beyond the embolus 200. In other words, the second step is carried out such that the forward end 101 of the guide wire 10 passes through the gap between the embolus 200 and the inner wall of the vessel 100 and gets over the embolus 200. This operation can be easily accomplished by using the guide wire 10 (micro-guide wire) which has good lubricity.
[2] After the forward end 101 of the guide wire 10 has got over the embolus 200, the catheter 8 is advanced relative to the guide wire 10, so that the forward end of the catheter 8 gets in the gap between the embolus 200 and the inner wall of the vessel 100, as shown in Fig. 5. This operation is easy because the forward end of the catheter smoothly gets in the gap along the guide wire 10.
   According to the conventional therapy, the above-mentioned stage is followed by injection of a thrombolytic agent through the catheter 8. However, it is often experienced by doctors that there are emboli 200 which are not dissolved by a thrombolytic agent or dissolution by a thrombolytic agent takes a long time. The present invention is effective in such a case.
[3] The step shown in Fig. 5 is followed by the next step in which the guide wire 10 is removed and the wire 1A for removing an intravascular foreign body is inserted into the lumen 82 of the catheter 8.
[4] As shown in Fig. 6, the forward end of the coil 266 (the forward end 24) of the wire body 2 is projected from the forward open end 81 of the catheter 8. As the result, the capturing part 11A, which has been in the catheter 8 in its contracted shape, expands to form the foreign body capturing space 3 in which the embolus 200 is to be captured.
[5] Then, from the state shown in Fig. 6, the catheter 8 is slightly moved toward the base end and the forward end of the catheter 8 is retreated to the back end of the embolus 200. Now, the embolus 200 is captured (scooped) by the foreign body capturing space 3 of the capturing part 11A, as shown in Fig. 7. In other words, the embolus 200 enters the foreign body capturing space 3 from the upper side shown in Figs. 6 and 7. At this time, the embolus 200 is tightened up by the capturing part 11A.
[6] From the state shown in Fig. 7, the capturing part 11A is moved toward the base end by manipulating the manipulating member 273 of the linear body 27, so that the embolus 200 (or the capturing part 11A) approaches the auxiliary wire 7A. The capturing part 11A is moved further toward the base end, so that the auxiliary wire 7A stabs the embolus 200, as shown in Fig. 8.
[7] The wire 1A for removing an intravascular foreign body and the catheter 8 are removed all together, with the state shown in Fig. 8 maintained. Thus, the embolus 200 is collected in the guiding catheter or sheath introducer (not shown).

### Second Embodiment

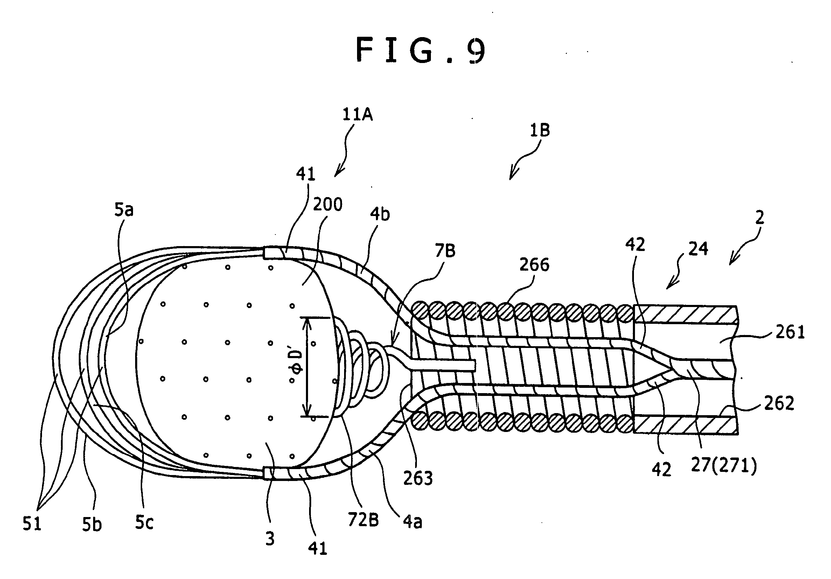

Fig. 9 is a plan view (with a partial longitudinal sectional view) showing the vicinity of the capturing part of the wire for removing an intravascular foreign body (in the first state) pertaining to the second embodiment of the present invention. In the following description, the right side and left side of Fig. 9 are designated as "base end (proximal end)" and "forward end (distal end)", respectively.

This figure will be referenced in the following description about the wire for removing an intravascular foreign body pertaining to the second embodiment of the present invention. Stress is placed on differences between the first embodiment and the second embodiment, and description of those items common to the two embodiments is omitted.

The wire for removing an intravascular foreign body pertaining to the second embodiment is identical with that pertaining to the first embodiment except for the shape of the auxiliary wire.

As shown in Fig. 9, the auxiliary wire 7B of the wire 1B for removing an intravascular foreign body has the expanded part 72B (which comes into contact with the embolus 200) in its first state. The expanded part 72B has a helical shape, with the loop diameter increasing in going toward the forward end.

The auxiliary wire 7B formed in this manner (in its first state) permits the embolus 200 to be held (or pressed down) between the expanded part 72B and the filaments 5a, 5b, and 5c. In this way, it is possible to surely capture and remove the embolus 200 in the vessel 100.

Being in a helical shape, the expanded part 72B holds the embolus 200 with a mild force without the possibility of breaking the embolus 200.

The outside diameter D' (φ D' in Fig. 9) at the forward end of the expanded part is not specifically restricted. It should preferably be 1 to 3 mm, and it should preferably be smaller by about 1 mm than the outside diameter (W) which is measures when the capturing part 11A is expanded.

### Third Embodiment

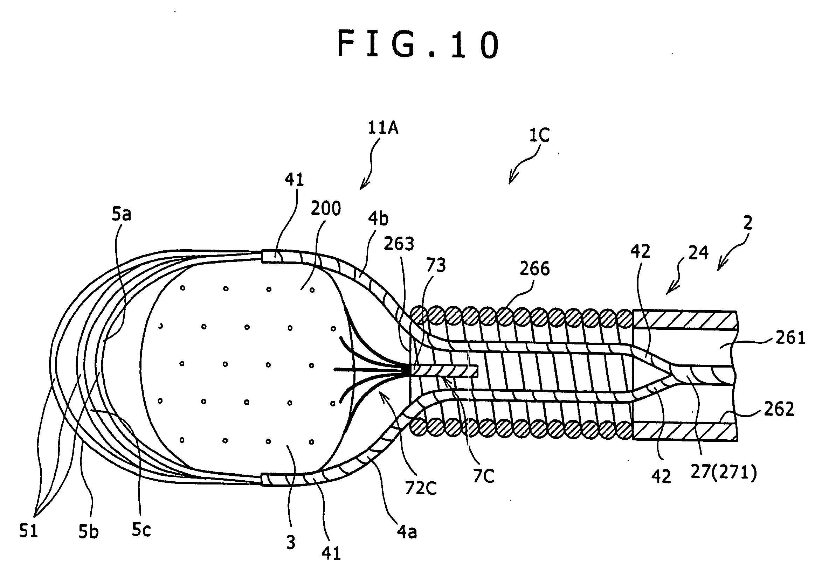

Fig. 10 is a plan view (with a partial longitudinal sectional view) showing the vicinity of the capturing part of the wire for removing an intravascular foreign body (in the first state) pertaining to the third embodiment of the present invention. In the following description the right side and left side of Fig. 10 are designated as "base end (proximal end)" and "forward end (distal end)", respectively.

This figure will be referenced in the following description about the wire for removing an intravascular foreign body pertaining to the third embodiment of the present invention. Stress is placed on differences from the above-mentioned embodiments, and description of those items common to them is omitted.

The wire for removing an intravascular foreign body pertaining to the second embodiment is identical with that pertaining to the first embodiment except for the shape of the auxiliary wire.

As shown in Fig. 10, the auxiliary wire 7C of the wire 1C for removing an intravascular foreign body has the expanded part 72C (which comes into contact with the embolus 200) in its first state. The expanded part 72C consists of a plurality (eight in this embodiment) of short linear bodies radially projecting to the forward end from the vicinity of the intermediate part 73.

The auxiliary wire 7C formed in this manner (in its first state) permits the embolus 200 to be held (or pressed down) between the expanded part 72C (forward ends of short linear bodies) and the filaments 5a, 5b, and 5c. In this way, it is possible to surely capture and remove the embolus 200 in the vessel 100.

Incidentally, the auxiliary wire 7C may be constructed such that four short linear bodies at the expanded part 72C stab the embolus 200.

### Fourth Embodiment

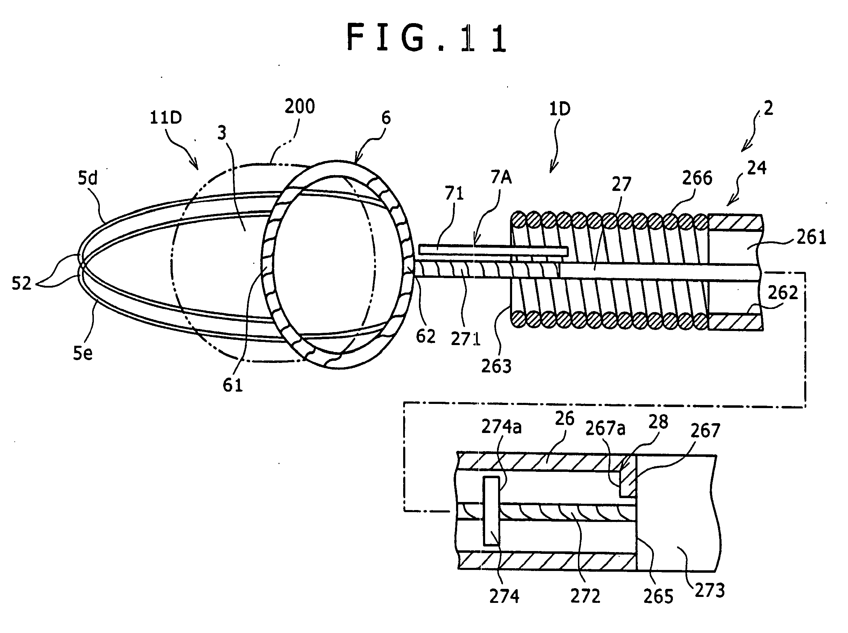

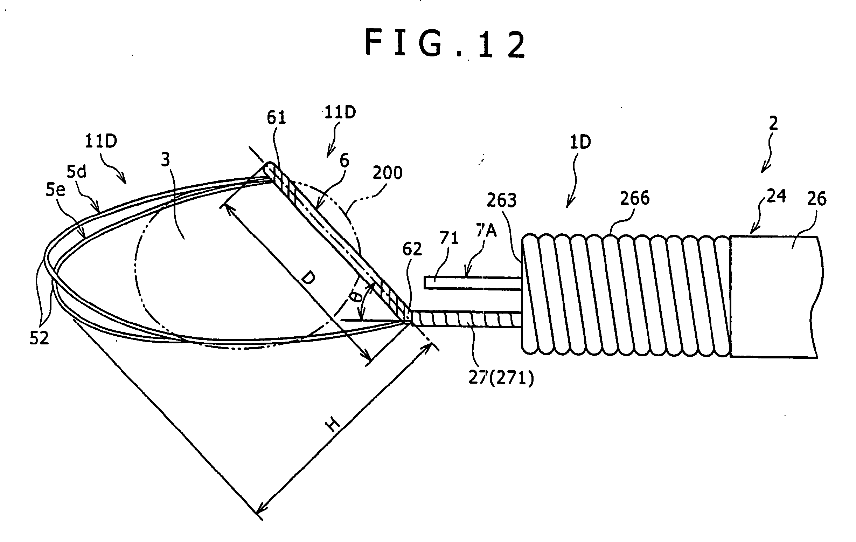

Fig. 11 is a plan view (with a partial longitudinal sectional view) showing the vicinity of the capturing part of the wire for removing an intravascular foreign body (in the first state) pertaining to the fourth embodiment of the present invention. Fig. 12 is a side view showing the wire for removing an intravascular foreign body which is shown in Fig. 11. In the following description the right side and left side of Figs. 11 and 12 are designated as "base end (proximal end)" and "forward end (distal end)", respectively.

The wire for removing an intravascular foreign body pertaining to the fourth embodiment is identical with that in the first embodiment except for the construction of the capturing part.

As shown in Figs. 11 and 12, the capturing part 11D of the wire 1D for removing an intravascular foreign body consists of the loop wire 6 and the filaments 5d and 5e. The loop wire 6 is arranged on the linear body 27 of the wire body (the forward end of the wire body 2). The filaments (two in this embodiment) are twisted with the loop wire 6.

As shown in Fig. 12, the loop wire 6 is bent in the same direction on the same plane, and it is inclined (fixed) with respect to the axis of the wire body 2.

The method for fixing the loop wire 6 to the linear body 27 (wire body 2) is not specifically restricted. Fixing may be accomplished by winding (braiding) the base end 62 of the loop wire 6 to the forward end 271 of the linear body 27 and then performing brazing, welding, or adhesion on them.

As shown in Fig. 12, the loop wire 6 in its natural state stands with respect to the linear body 27 (the wire body 2). (This state will be referred to as "standing state" hereinafter.) The angle of the loop wire 6 (θ in Fig. 12) with respect to the central axis of the linear body 27 should preferably be about 20 to 90 degrees, more preferably 40 to 60 degrees. This construction permits the forward end 61 of the loop wire 6 to get in the gap between the embolus 200 and the inner wall of the vessel 100 when the embolus 200 is to be captured. In this way it is possible to surely capture the embolus 200.

In the standing state, the loop diameter (D in Fig. 12) of the loop wire 4 is not specifically restricted; it should preferably be 1 to 20 mm, more preferably 2 to 5 mm.

As shown in Fig. 11, the filaments 5d and 5e are attached to the different parts of the loop wire 6 which are arranged symmetrically.

The filaments 5d and 5e are forwardly curved like an arch, and they cross each other at their arch tops 52. Crossing means that the filaments 5d and 5e are close to each other but does not necessarily mean that they are in contact with each other. They may be in contact with each other such that they move relative to each other.

In the standing state, the distance (H in Fig. 12) between the loop wire 6 and the top 52 is not specifically restricted. It should preferably be 3 to 20 mm, more preferably 3 to 10 mm.

The capturing part 11D constructed in this manner can surely capture (remove) the embolus 200.

Incidentally, the number of the filaments is not limited to two; it may be three or more.

The foregoing is about the wire for removing an intravascular foreign body and the medical instrument according to the present invention. It is not intended to restrict the scope of the present invention. The constituents of the wire and medical instrument may be modified or replaced by (or reinforced with) those which function in the same way.

Two or more of the above-mentioned embodiments may be combined to complete the wire for removing an intravascular foreign body.

The first embodiment may be modified such that the auxiliary wire is the one which is used in the second or third embodiment.

The fourth embodiment may be modified such that the auxiliary wire is the one which is used in the second or third embodiment.

The wire for removing an intravascular foreign body may be modified such that the auxiliary wire is moved relative to the capturing part rather than the capturing part is moved relative to the auxiliary wire.

In the third embodiment, the expanded part of the auxiliary wire is contractable and expandable; it may be constructed such that the expanded part is expandable when the auxiliary wire is in the first state.

The operation of the linear body of the wire proper is not limited to manipulation by the manipulating part. It will be accomplished by driving a rack and a pinion attached to the tube of the wire proper and the linear body, respectively. In this way, it is possible to easily manipulate the linear body.

The wire for removing an intravascular foreign body includes a flexible long wire body, a capturing part which is arranged on the forward end of the wire body and in which is formed a space to capture an intravascular foreign body, and an auxiliary wire which is arranged on the forward end of the wire body, the auxiliary wire assuming a first state in which it projects into the space and a second state in which it retreats from the space as it moves relative to the capturing part.

## Claims

1. A wire for removing an intravascular foreign body, comprising:
a flexible long wire body (2),
a capturing part (11A) which is arranged on the forward end (24) of the wire body and in which is formed a space for capturing an intravascular foreign body (200), and
an auxiliary wire (7A; 7B; 7C) which is arranged on the forward end of the wire body, said auxiliary wire assuming
a first state in which it projects into the space and is in contact with said foreign body (200) held between said auxiliary wire (7A; 7B; 7C) and said capturing part (11A) wherein said foreign body (200) is stabbed and/or held pressed by said auxiliary wire (7A; 7B; 7C) without breaking said foreign body (200), and
a second state in which it is out of the space and it's forward end (71) is directed towards said capturing part (11A) as it moves relative to the capturing part.

2. The wire for removing an intravascular foreign body as defined in Claim 1, in which the wire body (2) is composed of a tube (26) and a linear body (27) that passes through the tube for its movement relative to the tube, and in which the auxiliary wire (7A) is arranged on the forward end of the tube and the capturing part (11A) is arranged on the forward end of the linear body.

3. The wire for removing an intravascular foreign body as defined in Claim 1 or 2, in which the auxiliary wire (7A) is arranged on that part which is closer to the base end than the capturing part (11A).

4. The wire for removing an intravascular foreign body as defined in any of Claims 1 to 3, in which the auxiliary wire (7A) in the first state is in contact with the foreign body (200) and has an expanded part (72B) which expands toward the forward end.

5. The wire for removing an intravascular foreign body as defined in Claim 4, in which the expanded part (72B) assumes a helical shape.

6. The wire for removing an intravascular foreign body as defined in Claim 5, in which the loop diameter of the helical expanded part (72B) gradually increases in going toward the forward end.

7. The wire for removing an intravascular foreign body as defined in any of Claims 1 to 6, which has a means (28) for limiting the maximum extent to which the auxiliary wire (7A) projects in the first state.

8. A medical instrument which comprises the wire for removing an intravascular foreign body as defined in any of Claims 1 to 7 and a catheter having a lumen that receives therein the wire for removing an intravascular foreign body (200).

## Patentansprüche

1. Draht zum Entfernen eines intravaskulären Fremdkörpers mit:
einem flexiblen langen Drahtkörper (2),
einem Einfangteil (11A), das an dem vorderen Ende (24) des Drahtkörpers angeordnet ist und in dem ein Raum zum Einfangen eines intravaskulären Fremdkörpers (200) ausgebildet ist, und
einem Hilfsdraht (7B; 7B; 7C), der an dem vorderen Ende des Drahtkörpers angeordnet ist, wobei der Hilfsdraht
einen ersten Zustand einnimmt, in dem er in den Raum hinein vorragt, und mit dem Fremdkörper (200) in Kontakt steht, der zwischen dem Hilfsdraht (7A; 7B; 7C) und dem Einfangteil (11A) gehalten wird, wobei der Fremdkörper (200) durch den Hilfsdraht (7A; 7B; 7C) aufgestochen und / oder gedrückt gehalten wird, ohne dass der Fremdkörper (200) zerbricht, und
einen zweiten Zustand einnimmt, in dem er außerhalb des Raumes ist, und sein vorderes Ende (71) zu dem Einfangteil (11A) gerichtet ist, wenn er sich relativ zu dem Einfangteil bewegt.

2. Draht zum Entfernen eines intravaskulären Fremdkörpers gemäß Anspruch 1, wobei der Drahtkörper (2) aus einer Röhre (26) und einem linearen Körper (27) besteht, der durch die Röhre zum Zwecke seiner zu der Röhre erfolgenden Relativbewegung tritt, und wobei der Hilfsdraht (7A) an dem vorderen Ende der Röhre angeordnet ist und das Einfangteil (11A) an dem vorderen Ende des linearen Körpers angeordnet ist.

3. Draht zum Entfernen eines intravaskulären Fremdkörpers gemäß Anspruch 1 oder 2, wobei
der Hilfsdraht (7a) an dem Teil angeordnet ist, der näher zu dem Basisende als das Einfangteil (11A) ist.

4. Draht zum Entfernen eines intravaskulären Fremdkörpers gemäß Ansprüche 1 bis 3, wobei
der Hilfsdraht im ersten Zustand mit dem Fremdkörper (200) in Kontakt steht, und er einen erweiterten Teil (72B) hat, der zum vorderen Ende hin sich erweitert.

5. Draht zum Entfernen eines intravaskulären Fremdkörpers gemäß Anspruch 4, wobei
der erweiterte Teil (72B) eine spiralartige Form einnimmt.

6. Draht zum Entfernen eines intravaskulären Fremdkörpers gemäß Anspruch 5, wobei
der Schleifendurchmesser des spiralartigen erweiterten Teils (72B) zu dem vorderen Ende hin allmählich größer wird.

7. Draht zum Entfernen eines intravaskulären Fremdkörpers gemäß einem der Ansprüche 1 bis 6, der
eine Einrichtung (28) zum Begrenzen des maximalen Ausmaßes, bis zu dem der Hilfsdraht (7A) in dem ersten Zustand vorragt, hat.

8. Medizinisches Instrument mit
dem Draht zum Entfernen eines intravaskulären Fremdkörpers gemäß einem der Ansprüche 1 bis 7 und
einem Katheder mit einem Lumen, in dem der Draht zum Entfernen eines intravaskulären Fremdkörpers (200) aufgenommen ist.

## Revendications

1. Fil pour retirer un corps étranger intra-vasculaire, comprenant :
un corps long flexible (2) du fil,
une partie de capture (11A) qui est agencée sur l'extrémité avant (24) du corps du fil et dans laquelle un espace est formé pour capturer un corps étranger (200) intra-vasculaire,
et
un fil auxiliaire (7A; 7B; 7C) qui est agencé sur l'extrémité avant du corps du fil, ledit fil auxiliaire adoptant
un premier état dans lequel il se projette dans l'espace et se met en contact avec ledit corps étranger (200) maintenu entre ledit fil auxiliaire (7A; 7B; 7C) et ladite partie de capture (11A) où ledit corps étranger (200) est piqué et/ou maintenu pressé par ledit fil auxiliaire (7A; 7B; 7C) sans casser ledit corps étranger (200),
et
un deuxième état dans lequel il est en dehors de l'espace et son extrémité avant (71) est dirigée vers ladite partie de capture (11A) à mesure qu'il se déplace par rapport à la partie de capture.

2. Fil pour retirer un corps étranger intra-vasculaire tel que défini dans la revendication 1, dans lequel le corps (2) du fil est composé d'un tube (26) et d'un corps linéaire (27) qui passe par le tube pour son mouvement par rapport au tube, et dans lequel le fil auxiliaire (7A) est agencé sur l'extrémité avant du tube et la partie de capture (11A) est agencée sur l'extrémité avant du corps linéaire.

3. Fil pour retirer un corps étranger intra-vasculaire selon la revendication 1 ou 2, dans lequel le fil auxiliaire (7A) est agencé sur la partie qui est plus proche de l'extrémité de base que la partie de capture (11A).

4. Fil pour retirer un corps étranger intra-vasculaire selon l'une des revendications 1 à 3, dans lequel le fil auxiliaire (7A) dans le premier état est en contact avec le corps étranger (200) et a une partie agrandie (72B) qui s'agrandi vers l'extrémité avant.

5. Fil pour retirer un corps étranger intra-vasculaire selon la revendication 4, dans lequel la partie agrandie (72B) adopte une forme hélicoïdale.

6. Fil pour retirer un corps étranger intra-vasculaire selon la revendication 5, dans lequel le diamètre de boucle de la partie hélicoïdale agrandie (72B) augmente graduellement en allant vers l'extrémité avant.

7. Fil pour retirer un corps étranger intra-vasculaire selon l'une des revendications 1 à 6, qui dispose d'un moyen (28) qui limite l'ampleur maximale à laquelle le fil auxiliaire (7A) se projette dans le premier état.

8. Instrument médical qui comprend le fil pour retirer un corps étranger intra-vasculaire tel que défini dans l'une quelconque des revendications 1 à 7 et un cathéter ayant une lumière dans laquelle le fil est reçu afin de retirer un corps étranger (200) intra-vasculaire.
